# EUROPÄISCHE PATENTSCHRIFT

(11) **EP 0 572 845 B1**
(45) Veröffentlichungstag und Bekanntmachung des Hinweises auf die Patenterteilung: **01.04.1998**
(21) Anmeldenummer: 93107931.3
(22) Anmeldetag: 14.05.1993
(51) Int. Cl.: G01N 33/543, G01N 33/569

(54) **Verfahren zur immunchemischen Bestimmung eines Analyten**
Method for immunochemical measurement of an analyte
Méthode de mesure immunochimique d'un analyte

(30) Priorität: 03.06.1992 DE 4218257
(43) Veröffentlichungstag der Anmeldung: 08.12.1993
(73) Patentinhaber: Behring Diagnostics GmbH, 35001 Marburg (DE)
(72) Erfinder: Brust, Stefan, W-3550 Marburg-Michelbach (DE)

(56) Entgegenhaltungen:
- EP-A- 0 363 942
- EP-A- 0 379 216
- EP-A- 0 452 503

## Beschreibung

Die Erfindung betrifft ein Verfahren zur immunchemischen Bestimmung eines Analyten in einer Probe mittels eines ersten spezifischen Bindungspartners, wobei der spezifische Bindungspartner an einem Träger immobilisiert ist, und das Ausmaß der Bindung des Analyten an den spezifischen Bindungspartner mittels eines weiteren spezifischen Bindungspartners, der direkt oder indirekt eine Markierung trägt, bestimmt wird.

Übliche immunologische Verfahren zur Diagnose von Erkrankungen, die mit einer Bildung von spezifischen Antikörpern gegen einen Krankheitsauslöser, wie Viren, Bakterien, Allergene, Autoantigene oder bestimmte Arzneimittel einhergehen, beruhen auf der Fähigkeit dieser Antikörper zur Komplexbildung mit antigenen Strukturen des Auslösers.

Bei einigen dieser Verfahren, den allgemein als heterogener Immunoassay bezeichneten, wird eine auf den Gehalt z. B. an spezifischen Antikörpern (Analytantikörper) zu prüfende Probe mit antigenen Strukturen des Krankheitsauslösers in Kontakt gebracht, wobei diese antigenen Strukturen an geeigneten bekannten Trägermaterialien immobilisiert sind. In der Probe enthaltene Analytantikörper werden als Immunkomplex an die an dem Trägermaterial immobilisierten antigenen Strukturen des Krankheitsauslösers gebunden und nachgewiesen. Zum Nachweis können Nachweisantikörper bzw. andere spezifische Rezeptoren z. B. Protein A, die zur Komplexbildung mit dem Analytantikörper der Probe befähigt sind, verwendet werden.

Das Nachweisreagenz trägt in der Regel eine Markierung, welche die Menge des gebundenen spezifischen Antikörpers meßtechnisch erfaßbar macht.

Gebräuchliche Markierungen sind: Radioaktive Isotope, Enzyme, fluoreszierende, phosphoreszierende oder lumineszierende Substanzen, Substanzen mit stabilen ungepaarten Elektronen, Erythrozyten, Latexpartikel, Magnetpartikel, Metallsole.

Bei diesen Verfahren sind sowohl einstufige als auch mehrstufige Nachweismethoden bekannt. Jeder Verfahrensschritt wird üblicherweise mit einem Trennprozess (Waschschritt) beendet.

Die Etablierung besonders sensitiver Immunoassays ist bereits seit langem ein Anliegen vieler Entwicklungsgruppen. In der EP-A-0 379 216 beispielsweise wird ein sensitives Verfahren zur gleichzeitigen Bestimmung mehrerer verschiedener Analyten vorgeschlagen, indem unterschiedliche Rezeptoren der unterschiedlichen Analyten über einen konstanten Anteil an einen einheitlichen immobilisierten Bindungspartner gebunden werden. Das Nachweisprinzip der Analyten ist das des Sandwichimmunoassays.

In der EP-A-0 452 503 wird ein Festphasensandwichassay zum Nachweis von humanem Thrombomodulin vorgeschlagen, der sich durch die Verwendung eines monoklonalen und eines polyklonalen Antikörpers auszeichnet.

Die EP-A-0 363 942 offenbart Festphasenimmunoassays, bei denen der nachzuweisende Analyt mit Rezeptoren R1, R2 und R3 inkubiert wird, wobei R2 und R3 Analytkomponenten enthalten, R2 immobilisiert und R3 markiert ist. R1 kann spezifisch an den Analyten sowie die Analytkomponenten von R2 und R3 binden, so daß immobilisierte, markierte Immunkomplexe aus R2+R1+R3 entstehen können. Der Nachweis des Analyten erfolgt kompetitiv, indem er die Bildung dieses immobilisierten, markierten Immunkomplexes behindert. Der Vorteil des Verfahrens gemäß EP-A-0 363 942 soll in der Verwendung nicht modifizierter, gegebenenfalls polyklonaler Antikörper liegen.

Die sehr einfach durchzuführende Technik der Einschritt-Methode bei heterogenen Immunoassays ist allerdings nicht zum Nachweis aller Krankheitsmarker geeignet. Oft müssen aus technischen Gründen zwei- oder auch mehrstufige Verfahrensschritte angewandt werden.

Diese Methoden sind sehr spezifisch, haben allerdings den Nachteil, daß die nachzuweisenden Krankheitsauslöser oder dagegen gerichtete Antikörper, welche im ersten Verfahrensschritt mit dem immobilisierten, spezifischen Rezeptor einen Komplex eingegangen sind, in den darauffolgenden Inkubationsschritten in einer dem Fachmann bekannten Rückreaktion sich zum Teil wieder aus dem Komplex lösen können und sich somit der Nachweisreaktion entziehen, wodurch u. a. die Sensitivität stark eingeschränkt wird.

Die diagnostische Leistungsfähigkeit solcher mehrstufigen Verfahren wird immer dann besonders eingeschränkt, wenn die Rückreaktionsrate zwischen immobilisiertem Rezeptor und nachzuweisendem Agens hoch ist. Dies ist zum Beispiel bei niederaffinen Antikörpern gegen Krankheitsauslöser oder gegen Arzneimittel der Fall. Bekannt sind diese Probleme besonders auch bei Verfahren zum Nachweis von häufig mutierenden Krankheitsauslösern oder Krankheitsmarkern, welche mit dem immobilisierten, spezifischen Rezeptor nach Mutation eine geringere Wechselwirkung zeigen.

Daher bestand die Aufgabe Reagenzien zu finden, die die aufgezeigten Nachteile nicht aufweisen.

Überraschenderweise wurde festgestellt, daß die Rückreaktionsrate durch die Zugabe eines Bindefaktors gegen Strukturmerkmale des nachzuweisenden Agens erheblich reduziert wird. Dieser Bindefaktor muß mehr als eine bindungsfähige Stelle für das nachzuweisende Agens aufweisen und darf den immunchemischen Nachweis des Agens nicht stören.

Das nachzuweisende Agens (Analyt) im Sinne dieser Erfindung kann sowohl ein z. B. durch einen Krankheitsauslöser induzierter Antikörper als auch ein Antigen wie z. B. der Krankheitsauslöser selbst sein.

Gegenstand der Erfindung ist daher ein Verfahren zur immunologischen Bestimmung eines oder mehrerer Analyten mittels eines spezifischen Bindungspartners, wobei der spezifische Bindungspartner an einem Träger immobilisiert ist und das Ausmaß der Bindung des Analyten an den spezifischen Bindungspartner mittels eines weiteren spezifischen Bindungspartners, der direkt oder indirekt eine Markierung trägt, bestimmt wird, wobei in dem Verfahren zusätzlich ein Bindefaktor verwendet wird, welcher mehr als eine bindungsfähige Stelle für das nachzuweisende Agenz aufweist, der keine Affinität zu dem immobilisierten spezifischen Bindungspartner aufweist und der nicht markiert ist.

Nicht markiert heißt hierbei, daß er entweder kein oder zumindest nicht das Label trägt, mit dem das Ausmaß der Bindung des Analyten an den spezifischen Bindungspartner bestimmt wird.

Zugabe im Sinne dieser Erfindung bedeutet auch, daß der Bindefaktor bei den entsprechenden Verfahrensschritten an der Festphase immobilisiert ist oder bei den dem Fachmann bekannten matrixchemischen Nachweismitteln bereits auf der Phase eingetrocknet vorliegt.

Die Verfahren, in denen Bindefaktoren verwendet werden können sind in allen ihren Ausführungsformen an sich dem Fachmann bekannt. Es gibt dabei Einschritt- und Mehrschrittverfahren, wobei bei letzteren in der Regel zwischen den einzelnen Schritten ein Waschschritt eingesetzt wird.

Wichtig ist, daß das erfindungsgemäße Verfahren in geeigneter Fonn bei allen immunchemischen Verfahren, bei denen in einem ersten Schritt eine immunchemische oder vergleichbare Bindung eines Analyten an einen bevorzugterweise immobilisierten, spezifischen Bindungspartner erfolgt und in einem zweiten aber nicht unbedingt zeitlich getrennten Schritt ein direkter oder indirekter Nachweis erfolgt, eingesetzt werden kann.

Ohne damit eine bestimmte Wirkungsweise der Bindefaktoren zu postulieren, scheint es vorteilhaft, wenn der Analyt, der ein Peptid oder ein Protein sein kann, neben den Bindungsstellen für den spezifischen Festphasen- und Nachweisbindungspartner mindestens ein weiteres Epitop aufweist.

Bevorzugt werden die Bindefaktoren in den als Sandwich-ELISA dem Fachmann bekannten Verfahren eingesetzt, wobei bevorzugterweise als Markierungssystem ein Enzym, bevorzugterweise mit einem chromogenen oder fluorogenen Substrat oder ein chemolumineszentes Label verwendet wird. Die Ausführungsform des Nachweisverfahrens beeinflußt jedoch primär die Verwendungsmöglichkeiten der Bindefaktoren nicht.

Als Festphasen werden bevorzugterweise Mikrotitrationsplatten, Magnetpartikel, Latexpartikel oder matrixchemische Testelemente, wie z. B. Fasern oder Membranen enthaltende Testmodule verwendet.

Dem Fachmann ist auch bekannt, daß immunchemische Verfahren wie oben beschrieben zur gleichzeitigen Bestimmung von unterschiedlichen Analyten, wie z. B. HIV 1/2 oder HIV 1+2/HCV, eingesetzt werden können. Solche Ausführungsformen sind hiermit auch eingeschlossen.

Vorteilhaft sind Verfahren, bei denen der Bindefaktor bei dem Reaktionsschritt zugegeben wird, bei dem der Analyt an den zweiten, vorteilhafterweise markierten spezifischen Bindungspartner bindet.

Besonders vorteilhaft wirkt sich die Verwendung des Bindefaktors bei Mehrschrittverfahren aus, wobei der Bindefaktor bevorzugterweise nach dem ersten Waschschritt zugegeben wird.

Außerdem ist Gegenstand der Erfindung ein Reagenz zur Verwendung in einem vorstehend beschriebenen Verfahren, enthaltend einen an einem Träger immobilisierten ersten Bindungspartner und einen markierten zweiten Bindungspartner, wobei beide Bindungspartner für den nachzuweisenden Analyten spezifisch sind sowie einen für den Analyten spezifischen Bindefaktor, der mehr als eine Bindungsstelle für den Analyten besitzt, keine Affnität zu dem ersten spezifischen Bindungspartner aufweist, nicht mit besagter Markierung markiert ist und die Bindung des ersten und zweiten Bindungspartners an den Analyten nicht stört.

Desweiteren ist Gegenstand der Erfindung das obengenannte Verfahren, in dem der Bindefaktor ein Antikörperkonjugat ist.

Bindefaktoren im Sinne der Erfindung sind spezifische Bindungspartner, die mehr als eine bioaffine Bindungsstelle zum nachzuweisenden Agens aufweisen. Bindefaktor oder Komponenten dieses Bindefaktors können Konjugate der Antikörper sowie die Antikörper selbst darstellen. Antikörper im Sinne dieser Erfindung sind mono- und polyklonale Antikörper sowie die bekannten immunreaktiven Fragmente. Ebenfalls geeignet sind Lectine bzw. Konjugate mehrerer Lectine bzw. Konjugate von Lectinen mit Agens-spezifischen Antikörpern oder deren Fragmente.

Zugegeben werden kann der Bindefaktor zu jedem Zeitpunkt des jeweiligen Reaktionsschrittes der Bestimmung, vorteilhafterweise jedoch erfolgt die Zugabe nach der Bindung des Analyten an die Festphase.

Besonders bevorzugt als Bindefaktoren sind dabei Antikörper gegen die spezifischen Immunglobulinklasse des nachzuweisenden Antikörpers.

In dem Fall des Antigennachweises wird bevorzugterweise ein Antikörper als Bindefaktor verwendet, der nicht dasselbe Epitop erkennt wie der Festphasenantikörper oder der Konjugatantikörper.

Bevorzugt ist ein Verfahren bei dem mindestens ein Waschschritt verwendet wird.

Bevorzugterweise sind die Bindungsfaktoren nicht homolog zu den Anal-antikörpern. Wenn der Analytantikörper ein Humanantikörper ist, werden ganz besonders bevorzugt Maus- oder Kaninchenantikörper oder Antikörperkonjugate bzw. Konjugate aus Antikörperfragmenten.

Dem Fachmann geläufige Methoden zur Herstellung solcher Konjugate unter Erhalt ihrer bioaffinen Funktion sind beispielsweise die Verknüpfung durch chemische Reagenzien oder durch bioaffine Wechselwirkung.

Es können auch Hybridmoleküle durch chemische Synthese, die Hybridoma-Technik oder gentechnologischer Methoden erzeugt werden. Werden mehrere relevante Agenzien (z. B. Antikörper der Immunglobulinklassen G und M) gegen einen oder mehrere definierte Krankheitsauslöser z. B. gegen HIV 1 und HIV 2 nachgewiesen, kann der Bindefaktor beispielsweise gleichzeitig mit zwei oder mehr Agenzien bioaffin wechselwirken.

Das erfindungsgemäße Reagenz kann Verwendung finden in einer Vielzahl von Verfahren der Human- und Veterinärdiagnostik. Als Beispiele sollen angeführt werden, zwei- oder mehrstufige Tests zum Nachweis von Antikörpern verschiedener Immunglobulin-Klassen gegen Strukturmerkmale von Viren (z. B. Viren der Hepatitis A, B, C sowie verschiedener HIV-Typen), von bakteriellen und parasitären Erregern sowie von allergischen Erkrankungen. Weitere Beispiele sind der Nachweis von Krankheitserregern wie Viren (z. B. Hepatitis B-Virus), Bakterien, Parasiten oder Allergenen wie auch von Markern von Krankheiten (z. B. Tumormarker) in ein- und mehrstufigen Nachweisverfahren.

Die Erfindung wird durch das folgende Beispiel erläutert, ohne sie darauf einzuschränken:

### Beispiel

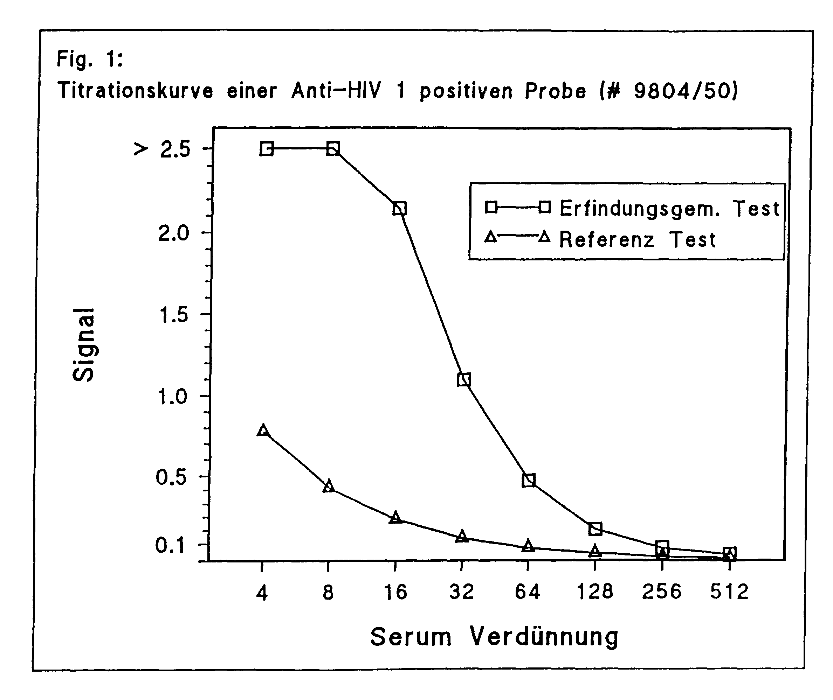

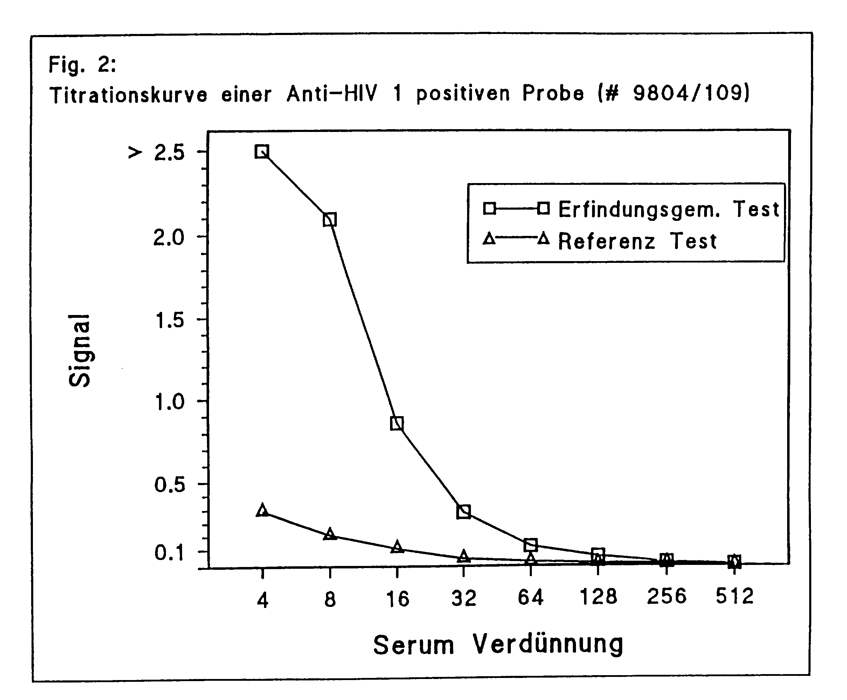

**a) Herstellen eines erfindungsgemäßen Reagenzes**
Zu 4 mg monoklonalem Anti-Human-IgG-Antikörper (Fc-spezifisch) in 1 ml PBS, pH 7,2 werden 1 ml 0.2 M Li-B03/20 % Dioxan gegeben und mit einem 15fach molaren Überschuß an N-y-Maleimidobutyrylsuccinimid (GMBS) versetzt und 1 h bei Raumtemperatur inkubiert. Das nicht umgesetzte heterobifunktionelle Reagenz wird über Gelchromatographie (Sephadex G-25) mit 0,1 molarem Na-Phosphat-Puffer + 5 mmol/l Nitrilotriessigsäure (NTA) pH 6.0 abgetrennt.
4 mg monoklonaler Anti-Human-IgG-Antikörper (Fc-spezifisch) in 4 ml 10 mmol/l Natriumphosphat, 100 mmol/l NaCl, pH 7.4 wird mit einem 24fach molaren Überschuß an N-Succinimidyl-3-(2-pyridyl-dithio)propionat (SPDP) 30 min. bei Raumtemperatur inkubiert, danach mit Di-thiothreitol (100fach molarer Überschuß gegenüber SPDP) 15 min. bei Raumtemperatur reduziert. Nach erfolgter Reduktion wird der nieder-molekulare Anteil über Gelfiltration an Sephadex G-25 (0,1 M Na-Phosphat, 5 mmol/l NTA, pH 6,0) entfernt.
Das SH-aktivierte Anti-Human-IgG wird mit dem Maleimid-aktivierten Anti-Human-IgG 2 Stunden bei Raumtemperatur inkubiert und anschließend mit 1/10 Vol 0.1 M N-Ethylmaleimid abgestoppt. Das Konjugat wird durch Gelchromatographie (ACA 34, LKB) mit 50 mmol/l TRIS/ HCl pH 7.4 gereinigt und anschließend auf 1 - 3 mg/ml ankonzentriert.
**b) Herstellung eines HIV 1 (env)-Peptid-Peroxidase-Konjugates**
10 mg HIV 1 (gp 41) Peptid (IAF BioChem, Kanada) werden in 1 ml Eisessig/Wasser (50 : 50, v/v) gelöst. Nach Neutralisieren mit 5 N Natronlauge wird mit einem 10fach molaren Überschuß an GMBS versetzt und 1 h bei Raumtemperatur inkubiert. Das nicht umgesetzte GMBS wird durch Gelfiltration (Sephadex G-25) mit 0.1 M Natriumphosphat/5 mmol/l NTA pH 6.0 abgetrennt.
10 mg Meerrettich-Peroxidase werden in 5 ml 10 mmol/l Natriumphosphat, 100 mmol/l NaCl, pH 8.0 mit 100fach molarem Überschuß an 2-Iminothiolan 1 h bei Raumtemperatur inkubiert. Anschließend wird freies Modifizierungsreagenz über Gelchromatographie (Sephadex G-25) mit 0.1 M Natriumphosphat/5 mmol/l NTA pH 6.0 entfernt.
Beide Eluate (SH-aktivierte Peroxidase und Maleimid-modifiziertes HIV 1-Peptid) werden vereinigt und über Nacht bei Raumtemperatur inkubiert. Nach Abstoppen mit 1/10 Vol 0.1 M N-Ethyl-Maleimid wird das Konjugat durch Gelchromatographie (Sephadex G-25) von nicht abreagiertem HIV 1-Peptid gereinigt. Nach Ankonzentrieren (2 mg/ml) wird das Peptid-Peroxidase-Konjugat bei -20°C gelagert.
**c) 2-Schritt-Enzymimmunoassay zum Nachweis von HIV 1-Antikörpern**
Ein Enzymimmunoassay zum Nachweis von Anti-HIV 1-Antikörpern wird wie folgt durchgeführt:
In den Vertiefungen einer mit HIV 1- und HIV 2-Peptiden beschichteten Testplatte (Enzygnost^{R} Anti HIV 1+2, Behringwerke AG, Marburg, FRG) werden 25 ml Probenpuffer (0,3 M Tris/HCl, 1 % Albumin, 2 % Tween 20, pH 7.2) mit 100 ml Human-Serum für 30 min. bei 37°C in-kubiert. Nach 4maligem Waschen mit 50 mmol/l PBS, 0,1 % Tween 20 werden 100 ml des nach Beispiel 1 b) hergestellten HIV 1-Peptid-Peroxidase-Konjugates (1:1000 in 0,1 M Tris/HCl, 1 % Albumin, 2 % Pluronic F 64, pH 8.1) einpipettiert.
Mit 4 weiteren Waschschritten wird die 30minütige Inkubation (+37°C) beendet. Die gebundene Peroxidase-Aktivität, die direkt mit der Anzahl an gebundenen HIV 1-spezifischen Antikörpern korreliert, wird durch Zugabe von H₂O₂/Tetramethylbenzidin (Behringwerke AG, Marburg, FRG) bestimmt.
**d) Anwendung des erfindungsgemäßen Reagenzes**
Anti-HIV 1 positive sowie Anti-HIV negative Seren werden im Enzymimmunoassay nach c) untersucht. In 10 ml Konjugatlösung werden zum einen 10 ml 50 mmol/l
Tris/HCl, pH 7.4 (Referenz-System) zum anderen 10 ml des erfindungsgemäßen nach a) hergestellten Reagenzes (erfindungsgemäßes System) zugegeben.
Die Resultate (Extinktionseinheiten) der Untersuchungen finden sich in der Tabelle sowie vergleichende Titrationen von HIV 1-Antikörper positiven Seren in Figur 1 und Figur 2.

**Tabelle**

| | Referenzsystem | Erfindungsgemäßes System |
|---|---|---|
| Kontrollserum, negativ | 0,019 | 0,018 |
| Kontrollserum, positiv | 0,964 | 1,681 |
| | 0,985 | 1,756 |
| | | |
| Anti-HIV neg. Seren | 0,015 | 0,019 |
| | 0,017 | 0,021 |
| | 0,032 | 0,028 |
| | 0,019 | 0,024 |
| | 0,030 | 0,018 |
| | | |
| Anti-HIV neg. Plasmen | 0,014 | 0,016 |
| | 0,017 | 0,016 |
| | 0,028 | 0,017 |
| | 0,016 | 0,016 |
| | 0,016 | 0,015 |
| | | |
| Anti HIV 1 pos. Plasma | > 2,500 | > 2,500 |
| 1 : 500 | > 2,500 | > 2,500 |
| 1 : 1000 | 1,424 | 2,440 |
| 1 : 2000 | 0,694 | 1,054 |
| 1 : 4000 | 0,317 | 0,478 |
| 1 : 8000 | 0,161 | 0,216 |
| 1 : 16.000 | 0,083 | 0,107 |
| 1 : 32.000 | 0,052 | 0,064 |
| 1 : 64.000 | 0,032 | 0,039 |
| | | |
| Grenzwert | 0,119 | 0,118 |
| | | |

## Patentansprüche

1. Verfahren zur immunchemischen Bestimmung eines Analyten in einer Probe mittels eines ersten spezifischen Bindungspartners, wobei dieser Bindungspartner an einem Träger immobilisiert ist und das Ausmaß der Bindung des Analyten an den ersten spezifischen Bindungspartner mittels eines zweiten spezifischen Bindungspartners, der direkt oder indirekt eine Markierung trägt, bestimmt wird, dadurch gekennzeichnet, daß zusätzlich ein Bindefaktor zugegeben wird, der mehr als eine bindungsfähige Stelle für den Analyten besitzt, keine Affinität zu dem ersten spezifischen Bindungspartner aufweist und nicht mit besagter Markierung markiert ist.

2. Verfahren nach Anspruch 1, wobei der Bindefaktor bei dem Reaktionsschritt zugegeben wird, bei dem der Analyt an den zweiten spezifischen Bindungspartner bindet.

3. Verfahren nach Anspruch 1, wobei der Analyt ein Antigen ist.

4. Verfahren nach Anspruch 1, wobei der Analyt ein Antikörper ist.

5. Verfahren nach einem der Ansprüche 1-4, dadurch gekennzeichnet, daß der Bindefaktor ein Antikörperkonjugat ist.

6. Reagenz zur Verwendung in einem Verfahren gemäß einem der vorhergehenden Ansprüche enthaltend einen an einem Träger immobilisierten ersten Bindungspartner und einen markierten zweiten Bindungspartner, wobei beide Bindungspartner für den nachzuweisenden Analyten spezifisch sind sowie einen für den Analyten spezifischen Bindefaktor, der mehr als eine Bindungsstelle für den Analyten besitzt, keine Affinität zu dem ersten spezifischen Bindungspartner aufweist, nicht mit besagter Markierung markiert ist und die Bindung des ersten und zweiten Bindungspartners an den Analyten nicht stört.

## Claims

1. A process for the immunochemical determination of an analyte in a sample by means of a first specific binding partner, this binding partner being immobilized on a support and the extent of the binding of the analyte to the first specific binding partner being determined by means of a second specific binding partner which directly or indirectly bears a label, which comprises additionally adding a binding factor which has more than one site which is capable of binding the analyte, possesses no affinity for the first specific binding partner, and is not labeled with said label.

2. The process as claimed in claim 1, wherein the binding factor is added in the reaction step in which the analyte binds to the second specific binding partner.

3. The process as claimed in claim 1, wherein the analyte is an antigen.

4. The process as claimed in claim 1, wherein the analyte is an antibody.

5. The process as claimed in one of claims 1-4, wherein the binding factor is an antibody conjugate.

6. A reagent for use in a process as claimed in one of the preceding claims, comprising a first binding partner which is immobilized on a support and a labeled second binding partner, both binding partners being specific for the analyte to be detected, and a binding factor which is specific for the analyte, has more than one binding site for the analyte, possesses no affinity for the first specific binding partner, is not labeled with said label, and does not disturb the binding of the first and second binding partner to the analyte.

## Revendications

1. Procédé pour la détermination immunochimique d'un analyte dans un échantillon, au moyen d'un premier partenaire de liaison spécique, ce partenaire de liaison spécifique étant immobilisé sur un support, et le degré de la liaison de l'analyte au premier partenaire de liaison spécifique étant déterminé au moyen d'un second partenaire de liaison spécifique qui porte directement ou indirectement un marqueur, caractérisé en ce que l'on ajoute en outre un facteur de liaison qui possède plus d'un site capable de liaison pour l'analyte, ne présente aucune affinité pour le premier partenaire de liaison spécifique et n'est pas marqué avec ledit marqueur.

2. Procédé selon la revendication 1, dans lequel le facteur de liaison est ajouté dans l'étape de réaction dans laquelle l'analyte se fixe au second partenaire de liaison spécifique.

3. Procédé selon la revendication 1, dans lequel l'analyte est un antigène.

4. Procédé selon la revendication 1, dans lequel l'analyte est un anticorps.

5. Procédé selon l'une des revendication 1 à 4, caractérisé en ce que le facteur de liaison est un conjugué d'anticorps.

6. Réactif pour utilisation dans un procédé selon l'une des revendications précédentes, contenant un premier partenaire de liaison immobilisé sur un support et un second partenaire de liaison marqué, les deux partenaires de liaison étant spécifiques de l'analyte à détecter, ainsi qu'un facteur de liaison spécifique de l'analyte, qui possède plus d'un site de liaison pour l'analyte, ne présente aucune affinité pour le premier partenaire de liaison spécifique, n'est pas marqué par ledit marqueur et ne gêne pas la fixation du premier partenaire de liaison et du second à l'analyte.
